Europäisches Patentamt
European Patent Office
Office européen des brevets

(19) (11) Publication number: **0 002 867 A1**

(12) **EUROPEAN PATENT APPLICATION**

(21) Application number: 78200378.4

(22) Date of filing: 18.12.78

(51) Int. Cl.²: C 07 C 103/85, A 61 K 31/165

(30) Priority: 27.12.77 US 864983

(43) Date of publication of application: 11.07.79 Bulletin 79/14

(84) Designated Contracting States: BE CH DE FR GB IT NL SE

(71) Applicant: SCHERICO LTD., Töpferstrasse 5, CH-6004 Lucerne (CH)

(72) Inventor: Neustadt, Bernard Ray, 24 Brook Place, West Orange New Jersey 07052 (US)
Inventor: Gold, Elijah Herman, 10 Roosevelt Avenue, West Orange New Jersey 07052 (US)

(74) Representative: Antony, Fritz, Dr. et al, P.O. Box 601 Winkelriedstrasse 35, CH-6002 Lucerne (CH)

(54) **5-(2-Anilinoalkylamino-1-hydroxyalkyl)salicylamides, their preparation and anti-hypertensive compositions containing them.**

(57) This invention relates to novel antihypertensive 5-(2-anilinoalkyl-amino-1-hydroxyalkyl)-salicylamides of the formula

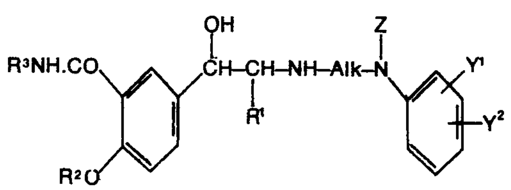

(I)

wherein

$R^1$ and $R^3$ are independently hydrogen atoms or lower alkyl groups;

$R^2$ is a hydrogen atom or a lower alkyl, loweralkoxyloweralkyl or hydroxyloweralkyl group;

Alk is an acyclic alkylene bridge containing 2–10 carbon atoms, with the proviso that there are 2–6 carbon atoms separating the nitrogen atoms that it bridges;

Z is a hydrogen atom or a lower alkyl, lower alkanoyl, lower alkylsulfonyl, arylsulfonyl, loweralkoxyloweralkyl, 2,2,2-trifluoroethyl or benzyl group;

and $Y^1$ and $Y^2$ are independently hydrogen or halogen atoms or hydroxy, trifluoromethyl, lower alkyl, lower alkoxy, nitro, unsubstituted amino, monoloweralkylamino, di-lower-alkylamino, loweralkanoylamino, loweralkylsulfonylamino, arylsulfonylamino, N-loweralkyl-N-loweralkanoylamino or N-loweralkyl-N-loweralkylsulfonylamino groups;

and the pharmaceutically acceptable acid addition salts thereof;

to their preparation and to pharmaceutical compositions containing them. The compounds can be used in the treatment of cardiovascular disorders, especially hypertension.

EP 0 002 867 A1

 0002867

"5-(2-Anilinoalkylamino-1-hydroxyalkyl)salicylamides, their preparation and anti-hypertensive compositions containing them"

The present invention relates to novel 5-(2-anilinoalkylamino-1-hydroxyalkyl)-salicylamides having valuable pharmacological properties, in particular antihypertensive activity, to processes for their preparation and to pharmaceutical compositions containing them.

The present invention therefore provides 5-(2-anilinoalkylamino-1-hydroxyalkyl)salicylamides of the formula

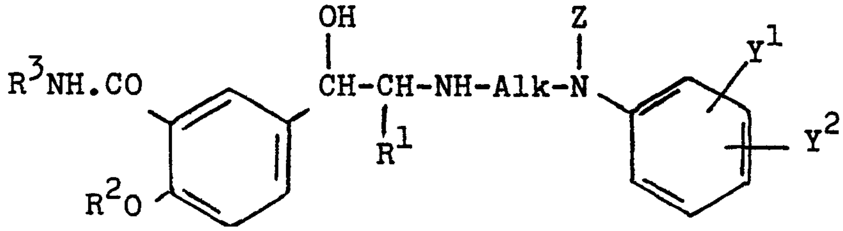

(I)

wherein $R^1$ and $R^3$ are independently hydrogen atoms or lower alkyl groups;

$R^2$ is a hydrogen atom or a lower alkyl, loweralkoxyloweralkyl, or hydroxyloweralkyl group;

Alk is an acyclic or cyclic alkylene bridge containing 2-10 carbon atoms, with the proviso that there are 2 - 6 carbon atoms separating the nitrogen atoms that it bridges;

Z is a hydrogen atom or a lower alkyl, lower alkanoyl, lower alkylsulfonyl, arylsulfonyl, loweralkoxyloweralkyl, 2,2,2-trifluoroethyl or benzyl group;

$Y^1$ and $Y^2$ are independently hydrogen or halogen atoms or hydroxy, trifluoromethyl, lower alkyl, lower alkoxy, nitro, amino, mono-loweralkylamino, di-loweralkylamino, loweralkanoylamino, loweralkylsulfonylamino, arylsulfonylamino, N-loweralkyl-N-loweralkanoylamino or N-loweralkyl-N-loweralkylsulfonylamino groups;

and the pharmaceutically acceptable acid addition salts thereof.

The lower alkyl groups referred to above contain 1-6 carbon atoms and are exemplified by methyl, ethyl, propyl, butyl, pentyl, hexyl and the corresponding branched

chain isomers thereof. The lower alkoxy groups likewise contain 1-6 carbon atoms and typically may be methoxy, ethoxy, n-propoxy, isopropoxy, n-butoxy, sec-butoxy and t-butoxy.

The lower alkanoyl groups referred to above contain 1-6 carbon atoms and are exemplified by formyl, acetyl, butyryl and valeryl.

The halogen atoms represented by $Y^1$ and $Y^2$ include fluorine, chlorine, bromine and iodine.

The Alk bridges having 2-6 carbon atoms separating the nitrogen atoms are represented by acyclic groups such as ethylene, trimethylene, tetramethylene and pentamethylene groups optionally substituted by lower alkyl groups. Thus, representative acyclic Alk groups of this invention are ethylene, 1-methylethylene, trimethylene, 2-methylethylene, 1-methyltrimethylene, tetramethylene, 1-methyltetramethylene, 1,1-dimethyltrimethylene, 1,2-dimethyltrimethylene, 1-ethyltrimethylene, 1-methyl-2-ethyltetramethylene, pentamethylene, and 1,1-dimethyl-3-methylpentamethylene. (The numbering of the Alk bridge commences with the carbon atom bound to the nitrogen atom of the ethanolamine residue in the 5-position of the

salicylamide moiety in formula I.)

A group of preferred compounds having an acyclic bridge Alk may be represented by the formula

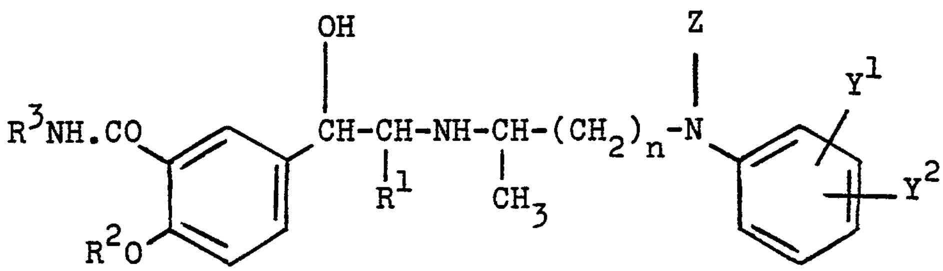 (II)

wherein n is 1-4, and $R^1$, $R^2$, $R^3$, Z, $Y^1$ and $Y^2$ are as hereinbefore defined. A particularly preferred bridge is of the formula

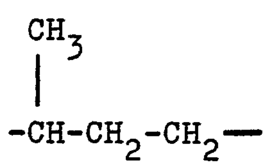 (III);

i.e., n is 2 in formula II.

A group of preferred compounds having a cyclic bridge Alk may be represented by the formula

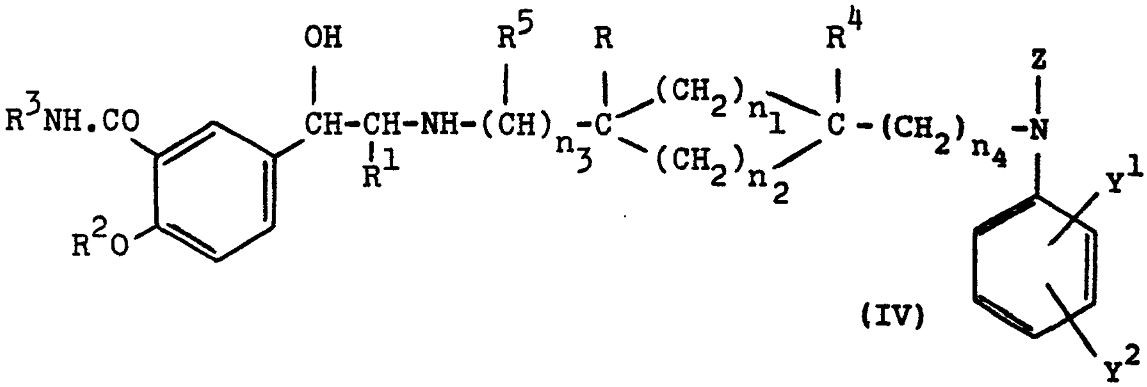

(IV)

wherein $\underline{n}_1$ is 0-2,

$\underline{n}_2$ is 1-5,

$\underline{n}_1 + \underline{n}_2$ is 2-6,

$\underline{n}_3$ and $\underline{n}_4$ are independently 0 or 1,

R, $R^4$ and $R^5$ are independently hydrogen atoms or lower alkyl groups with the proviso that the total number of carbon atoms in the Alk bridge does not exceed ten,

and $R^1$, $R^2$, $R^3$, Z, $Y^1$ and $Y^2$ are as hereinbefore defined.

There is a direct bond from the nitrogen atom to the carbon atom of the ring when $\underline{n}_3$ or $\underline{n}_4$ is zero, and a direct bond between the two ring carbon atoms carrying the radicals R and $R^4$ when $\underline{n}_1$ is zero.

Cyclic Alk bridges may have lower alkyl substituents and include 1,2-cyclopentylene, 1,3-cyclopentylene, 1,2-cyclohexylene, 1,3-cyclohexylene, 1,4-cyclohexylene, 1-methyl-1,4-cyclohexylene and 1,4-cycloheptylene, in their <u>trans</u>- and <u>cis</u>-forms. Cyclic Alk bridges that contain acyclic moieties include those of the following formulae (in their <u>cis</u>- and <u>trans</u>- forms):

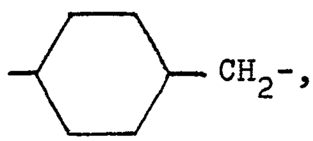

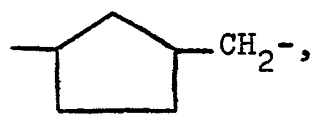

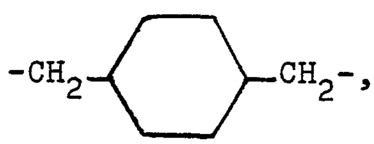

and

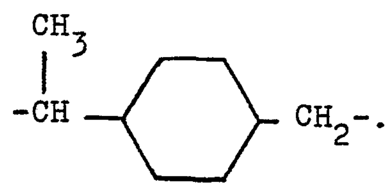

In a preferred groups of compounds of formula I, $R^2$ and $R^3$ are hydrogen atoms and $R^1$ is a methyl group or especially a hydrogen atom; $Y^1$ is a hydrogen atom, $Y^2$ is a methyl group or a hydrogen or halogen atom, Z is a hydrogen atom or a methyl or benzyl group and Alk is a trimethylene, tetramethylene or 1,4-cyclohexylene group (especially in the <u>trans</u>-form) or a group of the formula

$$-CH(CH_3).(CH_2)_n-$$

where n is 1-4.

Particularly preferred compounds of this invention are
5-{2-[3-(N-methylanilino)-2-propylamino]-1-hydroxyethyl} salicylamide;
5-{2-[4-(N-methylanilino)-2-butylamino]-1-hydroxyethyl} salicylamide;
5-{2-[5-(N-methylanilino)-2-pentylamino]-1-hydroxyethyl} salicylamide;
5-{2-[4-(4,N-dimethylanilino)-2-butylamino]-1-hydroxyethyl} salicylamide;
5-{2-[4-(4-fluoro-N-methylanilino)-2-butylamino]-1-hydroxyethyl} salicylamide; and
5-{2-[4-(4-chloro-N-methylanilino)-2-butylamino]-1-hydroxyethyl} salicylamide.

The pharmaceutically acceptable acid addition salts of the compounds of formula I may be derived from organic and inorganic acids such as sulfuric, phosphoric, hydrochloric, hydrobromic, sulfamic, citric, lactic, oleic, succinic, tartaric, cinnamic, acetic, benzoic, gluconic, ascorbic and related acids.

The compounds of formula I and their pharmaceutically acceptable acid addition salts can be prepared for

example by the following methods:

a) Reduction of a compound of the formula

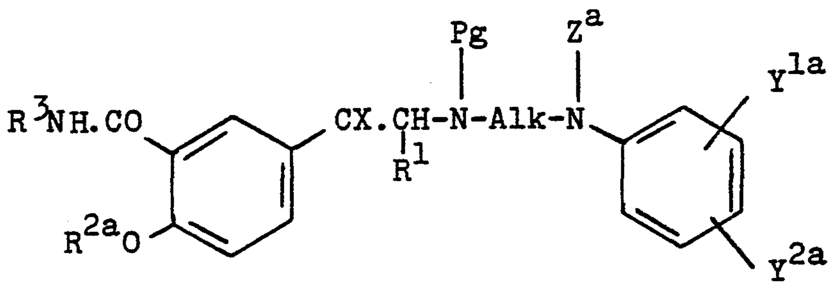

(V)

wherein X is an oxygen atom or (H,OH), Alk, $R^1$ and $R^3$ are as defined above, Pg is a hydrogen atom or a reductively removable protecting group, $R^{2a}$ and $Z^a$ are respectively $R^2$ and Z defined above or a reductively removable protecting group, and $Y^{1a}$ and $Y^{2a}$ are $Y^1$ and $Y^2$ above, or a hydroxy or amino group (including unsubstituted and mono-substituted amino groups, but excluding di-substituted amino groups, hereinbefore defined for $Y^1$ and $Y^2$) carrying a reductively removable protecting group;

provided that, when $R^{2a}$, Pg, $Z^a$, $Y^{1a}$ and $Y^{2a}$ are respectively the groups $R^2$, H, Z, $Y^1$ and $Y^2$, then X is an oxygen atom;
in an appropriate solvent.

The reduction of the group CX when X is an oxygen atom to the group CHOH can be effected by a number of reducing agents. The reducing agent is preferably a borohydride, e.g. lithium borohydride, potassium borohydride or especially sodium borohydride. The reduction is preferably carried out in a water-miscible alcohol as solvent, e.g., methanol, ethanol or isopropanol.

The group CX (when X is oxygen) can be reduced also by means of hydrogen and a catalyst, e.g. palladium, especially 5-10% Pd-C, in the presence of an organic solvent such as a lower alkanol, e.g. methanol or ethanol.

It is to be noted that many reduction processes, especially those using hydrogen and a catalyst, are not suitable for the preparation of compounds wherein $Y^1$ and/or $Y^2$ is a nitro group. Moreover, processes using hydrogen and a catalyst are not suitable for the preparation of compounds wherein Z is a benzyl group.

Reductively removable protecting groups that may be present in $Y^{1a}$ or $Y^{2a}$ or may be present as $R^{2a}$, Pg or $Z^a$ include benzyl, benzhydryl, trityl and benzyloxycarbonyl. These groups can all be removed by means of hydrogen and a catalyst, preferably as described above.

A particularly preferred embodiment of this process comprises the reduction of a compound of the formula

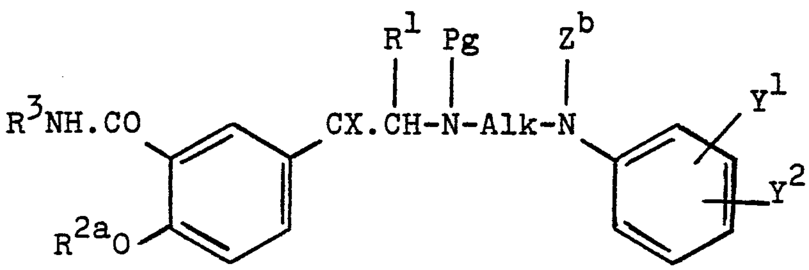

(VI)

wherein $R^1$, $R^{2a}$, $R^3$, Pg, Alk, X, $Y^1$ and $Y^2$ are as defined above, and $Z^b$ is the group Z defined above other than a benzyl group;

with the proviso that, when $R^{2a}$ and Pg are $R^2$ and a hydrogen atom respectively, then X is an oxygen atom. When X is an oxygen atom and $R^{2a}$ and/or Pg is a reductively removable protecting group, then this embodiment may be

effected in one step by means of hydrogen and a catalyst or in two steps by means of a borohydride and then hydrogen and a catalyst, the reaction conditions being as defined above. When more than one reductively removable group is present, all such groups are preferably identical, e.g., all benzyl groups or all benzyloxycarbonyl groups.

The compounds of the formula V or VI can be prepared by standard methods. For example, compounds of the formula VI can be prepared by condensation of a compound of the formula

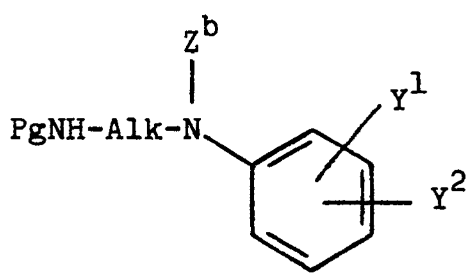

(VII)

with a compound of the formula

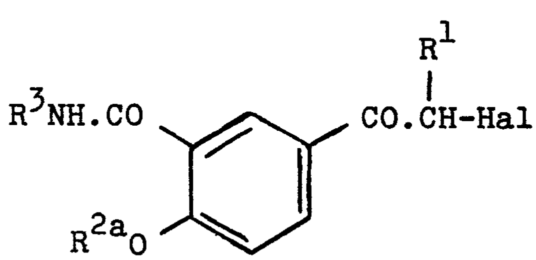

(VIII)

wherein $R^1$, $R^{2a}$, $R^3$, Alk, Pg, $Y^1$, $Y^2$ and $Z^b$ are as defined above and Hal is a chlorine or bromine atom. This condensation is effected in the presence of an acid acceptor and an organic solvent. An organic base, e.g. pyridine or triethylamine, can serve as solvent and acid acceptor. If a neutral solvent such as a dialkylamide, e.g. dimethylformamide, or alcohol is used, an inorganic base such as sodium or potassium carbonate can be used as acid acceptor.

By way of example, the following compounds in particular can be prepared by this process:

5-{2-[4-(N-methylanilino)-2-(2-methyl)butylamino]-1-hydroxyethyl} salicylamide;
5-{2-[<u>trans</u>-4-(N-methylanilino )-1-cyclohexylamino]-1-hydroxyethyl} salicylamide, m.p. 159-161°C.;
5-{2-[<u>cis</u>-4-(N-methylanilino)-1-cyclohexylamino]-1-hydroxyethyl} salicylamide;
5-{2-[<u>trans</u>-4-(N,4-dimethylanilino)-1-cyclohexylamino]-1-hydroxyethyl} salicylamide;
5-[2-(<u>trans</u>-4-anilino-1-cyclohexylamino)-1-hydroxyethyl] salicylamide;
5-{2-[<u>trans</u>-3-(N-methylanilino)-1-cyclohexylamino]-1-hydroxyethyl} salicylamide;

5-{2-[cis-3-(N-methylanilino)-1-cyclohexylamino]-1-hydroxyethyl} salicylamide;

5-{2-[trans-3-(N-methylanilino)-1-cyclopentylamino]-1-hydroxyethyl} salicylamide;

5-{2-[cis-3-(N-methylanilino)-1-cyclopentylamino]-1-hydroxyethyl} salicylamide;

5-{2-[trans-3-(N,4-dimethylanilino)-1-cyclopentylamino]--1-hydroxyethyl} salicylamide;

5-[2-(trans-3-anilino-1-cyclopentylamino)-1-hydroxyethyl]-salicylamide;

5-{2-[trans-3-(N-methylanilino)-1-cyclobutylamino]-1-hydroxyethyl} salicylamide;

5-{2-[cis-3-(N-methylanilino)-1-cyclobutylamino]-1-hydroxyethyl} salicylamide;

5-{2-[trans-3-(N,4-dimethylanilino)-1-cyclobutylamino]-1-hydroxyethyl} salicylamide;

5-[2-(trans-3-anilino-1-cyclobutylamino)-1-hydroxyethyl]salicylamide;

5-{2-[trans-2-(N-methylanilino)-1-cyclopentylamino]-1-hydroxyethyl} salicylamide;

5-{2-[cis-2-(N-methylanilino)-1-cyclopentylamino]-1-hydroxyethyl} salicylamide;

5-{2-[cis-4-(N-methylanilino)-1-cycloheptylamino]-1-hydroxyethyl} salicylamide;

5-{2-[trans-4-(N-methylanilino)-1-cycloheptylamino]-1-hydroxyethyl}salicylamide;

5-{2-[trans-2-(N-methylanilino)-1-cyclohexylamino]-1-hydroxyethyl}salicylamide;

5-{2-[cis-2-(N-methylanilino)-1-cyclohexylamino]-1-hydroxyethyl}salicylamide;

5-{2-[trans-2-(N-methylanilino)-1-methyl-1-r-cyclohexylamino]-1-hydroxyethyl}salicylamide;

5-{2-[cis-2-(N-methylanilino)-1-methyl-1-r-cyclohexylamino]-1-hydroxyethyl} salicylamide:

5-{2-[trans-4-(4-fluoro-N-methylanilino)-1-cyclohexylamino]-1-hydroxyethyl} salicylamide;

5-{2-[cis-4-(4-fluoro-N-methylanilino)-1-cyclohexylamino]-1-hydroxyethyl} salicylamide;

5-{2-[trans-4-(4-trifluoromethyl-N-methylanilino)-1-cyclohexylamino]-1-hydroxyethyl} salicylamide;

5-{2-[cis-4-(4-trifluoromethyl-N-methylanilino)-1-cyclohexylamino]-1-hydroxyethyl} salicylamide;

5-{2-[trans-4-(N-acetylanilino)-1-cyclohexylamino]-1-hydroxyethyl} salicylamide;

5-{2-[cis-4-(N-acetylanilino)-1-cyclohexylamino]-1-hydroxyethyl}salicylamide;

5-{2-[trans-4-(N-ethylanilino)-1-cyclohexylamino]-1-hydroxyethyl}salicylamide;

5-{2-[cis-4-(N-ethylanilino)-1-cyclohexylamino]-1-hydroxyethyl}salicylamide;

5-{2-[<u>trans</u>-4-(N-methanesulfonylanilino)-1-cyclohexylamino]-1-hydroxyethyl} salicylamide;

5-{2-[<u>cis</u>-4-(N-methanesulfonylanilino)-1-cyclohexylamino]-1-hydroxyethyl} salicylamide;

5-{2-[<u>cis</u>-4-(N-methylanilino)methyl-1-cyclohexylamino]-1-hydroxyethyl} salicylamide;

5-{2-[<u>trans</u>-4-(N-methylanilino)methyl-1-cyclohexylamino]-1-hydroxyethyl} salicylamide;

5-{2-[<u>cis</u>-3-(N-methylanilino)methyl-1-cyclohexylamino]-1-hydroxyethyl} salicylamide;

5-{2-[<u>trans</u>-3-(N-methylanilino)methyl-1-cyclohexylamino]-1-hydroxyethyl} salicylamide;

5-{2-[<u>cis</u>-2-(N-methylanilino)methyl-1-cyclohexylamino]-1-hydroxyethyl} salicylamide;

5-{2-[<u>trans</u>-2-(N-methylanilino)methyl-1-cyclohexylamino]-1-hydroxyethyl} salicylamide;

5-{2-[<u>cis</u>-3-(N-methylanilino)methyl-1-cyclopentylamino]-1-hydroxyethyl}salicylamide;

5-{2-[<u>trans</u>-3-(N-methylanilino)methyl-1-cyclopentylamino]-1-hydroxyethyl}salicylamide;

5-{2-[<u>cis</u>-2-(N-methylanilino)methyl-1-cyclopentylamino]-1-hydroxyethyl}salicylamide;

5-{2-[<u>trans</u>-2-(N-methylanilino)methyl-1-cyclopentylamino]-1-hydroxyethyl}salicylamide;

5-{2-[<u>cis</u>-3-(N-methylanilino)methyl-1-cyclobutylamino]-1-hydroxyethyl}salicylamide;

5-{2-[trans-3-(N-methylanilino)methyl-1-cyclobutylamino]-1-hydroxyethyl}salicylamide;

5-{2-[cis-4-(N-methylanilino)methyl-1-cycloheptylamino]-1-hydroxyethyl}salicylamide;

5-{2-[trans-4-(N-methylanilino)methyl-1-cycloheptylamino]-1-hydroxyethyl}salicylamide;

5-{2-[1-(cis-4-(N-methylanilino)methyl-1-cyclohexyl)ethylamino]-1-hydroxyethyl}salicylamide;

5-{2-[1-(trans-4-(N-methylanilino)methyl-1-cyclohexyl)ethylamino]-1-hydroxyethyl}salicylamide;

5-{2-[1-(cis-3-(N-methylanilino)methyl-1-cyclohexyl)ethylamino]-1-hydroxyethyl}salicylamide;

5-{2-[1-(trans-3-(N-methylanilino)methyl-1-cyclohexyl)ethylamino]-1-hydroxyethyl}salicylamide;

5-{2-[1-(cis-2-(N-methylanilino)methyl-1-cyclohexyl)ethylamino]-1-hydroxyethyl}salicylamide;

5-{2-[1-(trans-2-(N-methylanilino)methyl-1-cyclohexyl)ethylamino]-1-hydroxyethyl}salicylamide;

5-{ 2-[1-(cis-3-(N-methylanilino)methyl-1-cyclopentyl)ethylamino]-1-hydroxyethyl} salicylamide;

5-{2-[1-(trans-3-(N-methylanilino)methyl-1-cyclopentyl)-ethylamino]-1-hydroxyethyl}salicylamide;

5-{2-[1-(cis-2-(N-methylanilino)methyl-1-cyclopentyl)ethylamino]-1-hydroxyethyl}salicylamide;

5-{2-[1-(trans-2-(N-methylanilino)methyl-1-cyclopentyl)-ethylamino]-1-hydroxyethyl} salicylamide;

5-{2-[1-(cis-3-(N-methylanilino)methyl-1-cyclobutyl)ethylamino]-1-hydroxyethyl} salicylamide;

5-{2-[1-(trans-3-(N-methylanilino)methyl-1-cyclobutyl)ethylamino]-1-hydroxyethyl} salicylamide;

5-{2-[1-(cis-4-(N-methylanilino)methyl-1-cycloheptyl)ethylamino]-1-hydroxyethyl} salicylamide;

5-{2-[1-(trans-4-(N-methylanilino)methyl-1-cycloheptyl)ethylamino]-1-hydroxyethyl} salicylamide;

5-{2-[1-(cis-4-(N-methylanilino)-1-cyclohexyl)ethylamino]-1-hydroxyethyl} salicylamide;

5-{2-[1-(trans-4-(N-methylanilino)-1-cyclohexyl)ethylamino]-1-hydroxyethyl} salicylamide;

5-{2-[3-(N-methylanilino)-1-propylamino]-1-hydroxyethyl} salicylamide, m.p. 149-150°C.; and

5-{2-[4-(N-methylanilino)-butylamino]-1-hydroxyethyl} salicylamide, m.p. 156-158°C.

b) Compounds of the formula IA:

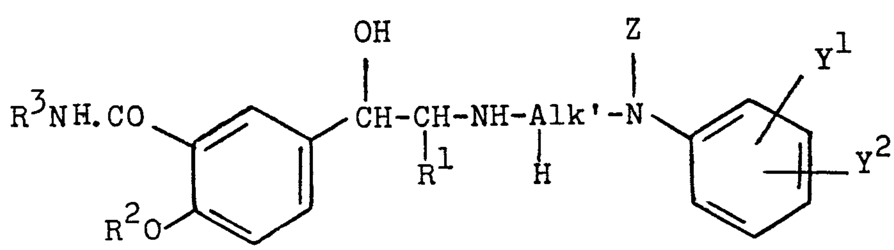

(IA)

wherein $R^1$, $R^2$, $R^3$, $Y^1$, $Y^2$ and Z are as defined above

and $\underset{\overset{|}{H}}{\text{Alk}'}$ is an acyclic or cyclic alkylene bridge as defined above for Alk with the proviso that the designated hydrogen atom in $\underset{\overset{|}{H}}{\text{Alk}'}$ and the nitrogen atom of the ethanolamine moiety $-CHOH.CHR^1-$ are bonded to the same carbon atom,

can be prepared by reductive condensation of a compound of the formula

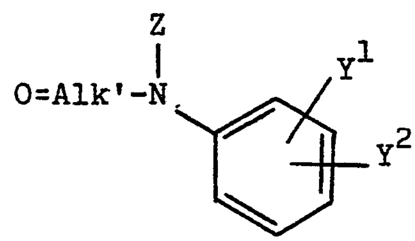

(IX)

wherein Alk', $Y^1$, $Y^2$ and Z are as defined above with a compound of the formula

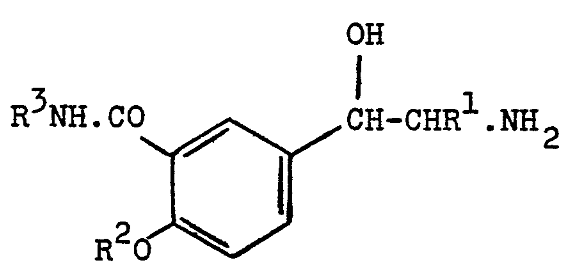

(X)

wherein $R^1$, $R^2$ and $R^3$ are as hereinbefore defined, in the presence of an organic solvent, preferably a lower alkanol such as methanol, ethanol or propanol. The reduction may be effected by means of catalytic hydrogenation (as described for process a)), or lithium thexyllimonyl borohydride, or 9-borobicyclo-[3,3,1]nonane, but preferably by means of sodium borohydride or sodium cyanoborohydride.

In a particularly preferred embodiment of this process, a compound of the formula II defined above is prepared by reductive condensation of a compound of the formula

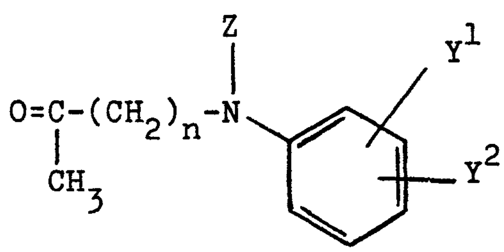

(IXA)

with a compound of the formula X, wherein $R^1$, $R^2$, $R^3$, $Y^1$, $Y^2$, Z and n are as defined for formula II. This process is preferred to process a) for the preparation of compounds of the formula II; in particular, the following compounds can be prepared by this process:

5-{2-[5-(4-fluoro-N-methylanilino)-2-pentylamino]-1-hydroxyethyl}salicylamide, m.p. (quarter hydrate) 160-162°C.;

5-{2-[5-(N-benzylanilino)-2-pentylamino]-1-hydroxyethyl}salicylamide, m.p. (hemihydrate) 144-146°C.

5-{2-[4-(N-methylanilino)-2-butylamino]-1-hydroxyethyl} salicylamide, m.p. 150-152°C.;

5-{2-[4-(4,N-dimethylanilino)-2-butylamino]-1-hydroxyethyl} salicylamide, m.p. 123-126°C.;

5-{2-[4-(4-chloro-N-methylanilino)-2-butylamino]-1-hydroxyethyl}salicylamide, m.p. 127°C.;

5-{2-[4-(3-chloro-4-methoxy-N-methylanilino)-2-butylamino]-1-hydroxyethyl}salicylamide;

5-{2-[4-(4-fluoro-N-methylanilino)-2-butylamino]-1-hydroxyethyl}salicylamide;

5-{2-[4-(4-hydroxy-N-methylanilino)-2-butylamino]-1-hydroxyethyl}salicylamide;

5-{2-[4-(4-methoxy-N-methylanilino)-2-butylamino]-1-hydroxyethyl}salicylamide;

5-{2-[4-(4-nitro-N-methylanilino)-2-butylamino ]-1-hydroxyethyl}salicylamide;

5-{2-[4-(4-amino-N-methylanilino)-2-butylamino]-1-hydroxyethyl}salicylamide;

5-{2-[4-(4-acetamido-N-methylanilino)-2-butylamino]-1-hydroxyethyl}salicylamide;

5-{2-[4-(4-methylamino-N-methylanilino)-2-butylamino]-1-hydroxyethyl}salicylamide;

5-{2-[4-(4-methanesulfonamido-N-methylanilino)-2-butylamino]-1-hydroxyethyl}salicylamide;

5-{2-[4-(4-trifluoromethyl-N-methylanilino)-2-butylamino]-1-hydroxyethyl}salicylamide;

5-{2-[4-(3-trifluoromethyl-N-methylanilino)-2-butylamino]-1-hydroxyethyl}salicylamide;

5-{2-[4-(3-methoxy-N-methylanilino)-2-butylamino]-1-hydroxyethyl}salicylamide;

5-{2-[4-(3-hydroxy-N-methylanilino)-2-butylamino]-1-hydroxyethyl}salicylamide;

5-{2-[4-(2,N-dimethylanilino)-2-butylamino]-1-hydroxyethyl}salicylamide;

5-{2-[4-(2-chloro-N-methylanilino)-2-butylamino]-1-hydroxyethyl}salicylamide;

5-{2-[4-(4-ethoxy-N-methylanilino)-2-butylamino]-1-hydroxyethyl}salicylamide;

5-{2-[4-(4-isopropyl-N-methylanilino)-2-butylamino]-1-hydroxyethyl}salicylamide;

5-{2-[4-(N-methylanilino)-2-butylamino]-1-hydroxy-2-methylethyl}salicylamide;

5-[2-(4-anilino-2-butylamino)-1-hydroxyethyl]salicylamide;

5-{2-[4-(N-methanesulfonylanilino)-2-butylamino]-1-hydroxyethyl}salicylamide;

5-{2-[4-(N-acetylanilino)-2-butylamino]-1-hydroxyethyl} salicylamide;
5-{2-[4-(N-(2,2,2-trifluoroethyl)anilino)-2-butylamino]-1-hydroxyethyl}salicylamide;
5-{2-[4-(N-ethylanilino)-2-butylamino]-1-hydroxyethyl} salicylamide;
5-{2-[3-(N-methylanilino)-2-propylamino]-1-hydroxyethyl}salicylamide, m.p. 143-145°C.;
5-{2-[3-(N,4-dimethylanilino)-2-propylamino]-1-hydroxyethyl}salicylamide;
5-[2-(3-anilino-2-propylamino)-1-hydroxyethyl]salicylamide;
5-{2-[5-(N-methylanilino)-2-pentylamino]-1-hydroxyethyl}salicylamide, m.p. 155-157°C.;
5-{2-[5-(N,4-dimethylanilino)-2-pentylamino]-1-hydroxyethyl}salicylamide;
5-[2-(5-anilino-2-pentylamino)-1-hydroxyethyl]salicylamide, m.p. 138-140°C.; 5-{2-[6-(N-methylanilino)-2-hexylamino]-1-hydroxyethyl}salicylamide, m.p. 157-160°C.;
5-{2-[6-(N,4-dimethylanilino)-2-hexylamino]-1-hydroxyethyl}salicylamide;
5-[2-(6-anilino-2-hexylamino)-1-hydroxyethyl]salicyamide;
5-{2-(4-(N-methylanilino)-2-(3-methyl)butylamino]-1-hydroxyethyl}salicylamide;
5-{2-[4-(N-methylanilino)-2-pentylamino]-1-hydroxyethyl} salicylamide;
5-{2-[3-(N-methylanilino)-2-butylamino]-1-hydroxyethyl}

salicylamide;

2-methoxy-5-{2-[4-N-methylanilino)-2-butylamino]-1-hydroxyethyl}benzamide;

2-(2-ethoxy)ethoxy-5-{2-[4-(N-methylanilino)-2-butylamino]-1-hydroxyethyl}benzamide;

2-(2-hydroxy)ethoxy-5-{2-[4-(N-methylanilino)-2-butylamino]-1-hydroxyethyl}benzamide;

5-{2-[4-(N-methylanilino)-2-butylamino]-1-hydroxyethyl}-N-methylsalicylamide; and

5-{2-[4-(N-methylanilino)-2-butylamino]-1-hydroxyethyl}-N-ethylsalicylamide.

The compounds of the formula I can be isolated from the above processes as the free bases or as their pharmaceutically acceptable acid addition salts.

The compounds of this invention possess one or more asymmetric carbon atoms and are produced by the above processes as stereoisomeric mixtures. The compounds may be used as such mixtures or separated into their enantiomeric and diastereoisomeric pure forms using conventional methods for the separation of such mixtures, such as fractional crystallization and chromatography e.g. on silica gel. In addition, a desired diastereoisomer may be obtained by synthesis using pure chiral components.

The following Examples illustrate the present invention. Some of the anilino-alkanones used as starting materials in the Examples were prepared according to Craig *et al.*, *J. Org. Chem.*, *29*, 410 (1964).

## EXAMPLE 1

**5-{2-[4-(N-Methylanilino)-2-butylamino]-1-hydroxyethyl} salicylamide**

To a solution of 3.92 g (20 mmol) 5-(2-amino-1-hydroxyethyl)-salicylamide and 3.54 g (20 mmol) 4-(N-methylanilino)-2-butanone in 100 ml ethanol add 1.26 g (20 mmol) of sodium cyanoborohydride. Stir 16 hrs., concentrate, and partition between ethyl acetate and 1N sodium bicarbonate solution. Dry and concentrate the organic layer. Recrystallize the residue from methanol to give a white solid, m.p. 150-152°C.

## EXAMPLE 2

**5-{2-[3-(N-Methylanilino)-2-propylamino]-1-hydroxyethyl} salicylamide**

To a solution of 20 mmol 5-(2-amino-1-hydroxyethyl)-salicylamide and 3.26 g (20 mmol) N-methylanilino-acetone in 100 ml methanol add 2.51 g (40 mmol) of sodium cyanoborohydride. Stir 16 hrs., concentrate, and partition between ethyl acetate and 1N sodium bicarbonate solution. Dry and concentrate the organic layer. Dissolve the resulting foam in chloroform and wash with

water. Filter off the solid which forms and recrystallize it from ethyl acetate to give a white solid, m.p. 143-145°C.

EXAMPLE 3

5-{2-[5-(N-Methylanilino)-2-pentylamino]-1-hydroxyethyl}salicylamide

(a) 5-(N-Methylanilino)-2-pentanone

To 49.4g (0.30 mol) 5-chloro-2-pentanone ethylene ketal and 64.2 g (0.60 mol) N-methylaniline in 100 ml toluene add 27.7 g (0.33 mol) sodium bicarbonate and 2.0 g potassium iodide. Reflux with a water separator overnight. Allow to cool, filter, and concentrate. Distil at 50°C./0.1 mm to remove N-methylaniline. Stir the residual liquid with 175 ml 1N hydrochloric acid for 2 hrs. Neutralize with sodium bicarbonate and extract with ether. Dry, concentrate, and distil to collect a fraction of b.p. 103-110°C./0.1 mm.

b) 5- 2-[5-(N-Methylanilino)-2-pentylamino]-1-hydroxyethyl salicylamide

Combine the above ketone (4.3 g; 21 mmol) with 20 mmol 5-(2-amino-1-hydroxyethyl)salicylamide and 2.0g 10% Pd/C in 200 ml methanol. Hydrogenate at 4.2 kg. cm.$^{-2}$ for 20 hrs. Filter off the catalyst, concentrate, and

partition the residue between ethyl acetate and 1N sodium bicarbonate solution. The organic layer deposits a solid. Filter it off and recrystallize it from methanol to give a white solid, m.p. 155-157°C.

EXAMPLE 4

5-{2-[4-(4-Chloro-N-methylanilino)-2-butylamino]-1-hydroxyethyl} salicylamide

To a solution of 20 mmol 5-(2-amino-1-hydroxyethyl)-salicylamide and 4.22 g (20 mmol) 4-(4-chloro-N-methyl-anilino)-2-butanone (m.p. 60-61°C.) in 100 ml methanol add 2.52 g (40 mmol) of sodium cyanoborohydride. Stir 6 days, concentrate, and partition between ethyl acetate and 1N sodium bicarbonate solution. Wash the organic layer with 1N hydrochloric acid. Neutralize the aqueous portion with sodium bicarbonate and extract with ethyl acetate. Dry, concentrate, and recrystallize the residue from ethyl acetate-hexane to give a white solid, m.p. 127-129°C.

EXAMPLE 5

5-{2-[4-(4,N-Dimethylanilino)-2-Butylamino]-1-Hydroxyethyl}Salicylamide

To a solution of 20 mmol 5-(2-amino-1-hydroxyethyl)-salicylamide and 5.7 g (30 mmol) 4-(N,4-dimethylanilino)-2-butanone (b.p. 93-6°C./0.1 mm) in 150 ml methanol,

add 1.9 g. (30 mmol) of sodium cyanoborohydride. After 20 hrs., concentrate, and partition between ethyl acetate and 1N sodium bicarbonate solution. Dry and concentrate. Warm the foam with ethyl acetate to give a white solid, m.p. 123-126°C.

EXAMPLE 6

5-{2-[6-(N-Methylanilino)-2-hexylamino]-1-hydroxyethyl}salicylamide

(a) 2-Benzyloxy-5-{2-[N-benzyl-6-(N-methylanilino)-2-hexylamino]-1-hydroxyethyl}benzamide

To N-(5-benzylaminohexyl)-N-methylaniline (3.06 g = 10 mmol) and 3.48 g (10 mmol) 2-benzyloxy-5-bromoacetyl-benzamide in 35 ml dimethylformamide, add 2.70 g (20 mmol) potassium carbonate. Stir 20 hrs. and pour into 150 ml water. Allow the gum to settle, decant, and dissolve the gum in ether. Extract with 1N hydrochloric acid, and then neutralize with sodium bicarbonate and extract with ethyl acetate. Dry and concentrate, and dissolve the oil in 50 ml ethanol. Add 0.3 g (8 mmol) sodium borohydride, stir 20 hrs., and concentrate. Partition between ethyl acetate and 1N sodium bicarbonate. Dry the organic layer and concentrate it to give a yellow foam.

b) 5-{2-[6-(N-Methylanilino)-2-hexylamino]-1-hydroxyethyl}salicylamide

Add the above amine to 250 ml ethanol containing 1.0 g 5% Pd/C. Hydrogenate at 4.2 kg. $cm.^{-2}$ for 20 hrs., filter and concentrate. Recrystallize the tacky solid from methanol to give a white solid, mp 157-160°C.

The compounds of the present invention are useful in the treatment of cardiovascular disorders and particularly in the treatment of mammalian hypertension. They are preferably administered orally but can also be administered by injection. Laboratory tests indicate that the effective dose ($ED_{50}$) by oral administration for a compound of the present invention will typically lie within the range of 0.05 to 10 mg/kg of mammalian weight.

The invention therefore provides pharmaceutical compositions containing as active ingredient one or more compounds of the formula I or pharmaceutically acceptable acid addition salts thereof, together with a pharmaceutical carrier or excipient. The compositions are preferably in the form of dosage units, e.g. tablets, capsules, or injectable preparations in ampoules. The compositions may also be in the form of syrups, elixirs or suspensions. The required daily dosage may be administered in single or divided doses. The exact dose to be administered will, of course, be dependent upon various factors such as the particular compound employed, age and weight of the subject mammal and the individual response. Dosage units preferably contain from 2 to 500 mg., more preferably from 25 to 250 mg., of active ingredient of the formula I (or pharmaceutically acceptable acid addition salt thereof).

Typical pharmaceutically acceptable carriers for use in formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; vegetable oils such as peanut oil, cottonseed oil, sesame oil, olive oil and corn oil; non-ionic, cationic and anionic surfactants; ethylene glycol polymers; β-cyclodextrin; fatty alcohols; hydrolyzed cereal solids; and other non-toxic compatible fillers, binders, disintegrants,

and lubricants commonly used in pharmaceutical formulations.

In treating certain patients with the compounds of this invention, it may be desirable to include other pharmaceutically active ingredients in the same composition. For example, in treating patients in whom salt and water retention is a problem, effective amounts of a diuretic, e.g., hydrochlorothiazide or trichloromethiazide, may be included.

Pharmaceutical Formulations

In the following examples, the active ingredient is preferably 5-{2-[5-(N-methylanilino)-2-butylamino]-1-hydroxyethyl} salicylamide or a pharmaceutically acceptable acid addition salt thereof, but an equivalent quantity of another compound (or more than one compound) of formula I, especially a compound named herein, may be substituted:

| Injectable Solution: | mg/ml |
|---|---|
| Active ingredient | 5.00 |
| Methyl p-hydroxybenzoate | 0.80 |
| Propyl p-hydroxybenzoate | 0.10 |
| Disodium Edetate | 0.10 |
| Citric Acid Monohydrate | 0.08 |

| Injectable Solution (continued) | mg/ml |
| --- | --- |
| Dextrose | 40.00 |
| Water for injection qs ad | 1.0 ml. |

Manufacturing Procedure:

Dissolve the p-hydroxybenzoates in a portion of water for injection at 60-70°C., and cool the solution to 25-35°C. Charge and dissolve all other excipients and the active ingredient. Bring the solution to final volume, filter it through a sterilizing membrane and fill into sterile containers.

Oral Formulations:

a) Capsules:

| Formula | Quantities per capsule (mg) | (mg) |
| --- | --- | --- |
| Active ingredient | 250.0 | 125.0 |
| Lactose | 173.0 | 86.5 |
| Corn Starch | 75.0 | 37.5 |
| Magnesium Stearate | 2.0 | 1.0 |
| | 500.C | 250.0 |

Manufacturing Procedure:

Blend the active ingredient, lactose and corn starch until uniform; then blend the magnesium stearate into the resulting powder. Encapsulate the mixture

into suitably sized two-piece hard gelatin capsules.

b) Tablets

| Formula | Quantities per tablet (mg) | (mg) |
| --- | --- | --- |
| Active ingredient | 250.0 | 125.0 |
| Lactose | 161.0 | 80.5 |
| Corn Starch | 12.0 | 6.0 |
| Water (per thousand tablets) | (120 ml evaporates) | (60 ml evaporates) |
| Corn Starch | 75.0 | 37.5 |
| Magnesium Stearate | 2.0 | 1.0 |
| | 500.0 | 250.0 |

Manufacturing Procedure:

Blend the active ingredient with the lactose until uniform. Blend the smaller quantity of corn starch with the water and add the resulting corn starch paste, then mix until a uniform wet mass is formed. Add the remaining corn starch to the resulting wet mass and mix until uniform granules are obtained. Screen the granules through a suitable milling machine, using a 3/4" stainless steel screen. Dry the milled granules in a suitable drying oven until the desired moisture content is obtained. Mill the dried granules through a suitable milling machine using 16 mesh stainless steel screen. Blend in the

magnesium stearate and compress the resulting mixture into tablets of desired shape, thickness, hardness and disintegration.

The compounds of the invention have useful antihypertensive activity as evidenced by tests in the spontaneously hypertensive rat (see the Table on the next page). Moreover, the compounds are substantially non-toxic at therapeutic doses.

In appropriate tests, the first, third and fourth compounds in the accompanying Table have shown little or no sympathetic inhibition at their antihypertensive doses.

Activity of compounds of Case 2149

| Name | Substituents (in relation to Formula II) $R^1=R^2=R^3=Y^1=H$ | | | Activity at mpK | | |
|---|---|---|---|---|---|---|
| | $Y^2$ | Z | n | 100 | 50 | 25 |
| 5-{2-[4-(N-methylanilino)-2-butylamino]-1-hydroxyethyl}salicylamide | H | $CH_3$ | 2 | +++ | ++ | |
| 5-{2-[4-(4,N-dimethylanilino)-2- butylamino]-1-hydroxyethyl}salicylamide | p-$CH_3$ | $CH_3$ | 2 | | | ++ |
| 5-{2-[6-(N-methylanilino)-2-hexylamino]-1-hydroxyethyl}salicylamide | H | $CH_3$ | 4 | | | ++ |

| Name | Substituents (in relation to Formula I) $R^1=R^2=R^3=Y^1=H$ | | | Activity at mpK | | |
|---|---|---|---|---|---|---|
| | $Y^2$ | Z | Alk | 100 | 50 | 25 |
| 5-{2-[trans-4-(N-methylanilino)-1-cyclohexylamino]-1-hydroxyethyl}salicylamide | H | $CH_3$ | 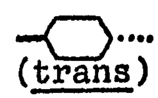 (trans) | | +++ | |

Activity key:

| | |
|---|---|
| +++ | Great |
| ++ | Moderate-Great |

0002867

Patent Claims

1. A compound of the formula

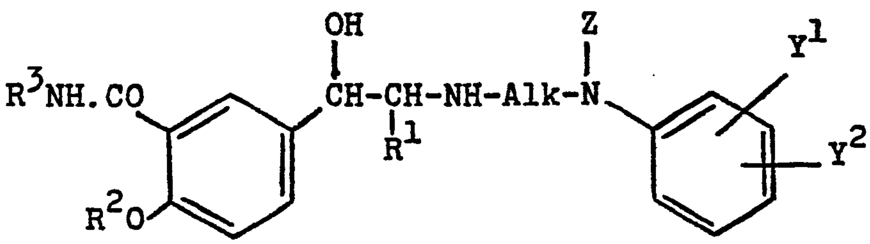

(I)

wherein $R^1$ and $R^3$ are independently hydrogen atoms or lower alkyl groups;

$R^2$ is a hydrogen atom or a lower alkyl, loweralkoxyloweralkyl or hydroxyloweralkyl group;

Alk is an acyclic or cyclic alkylene bridge containing 2-10 carbon atoms, with the proviso that there are 2 - 6 carbon atoms separating the nitrogen atoms that it bridges;

Z is a hydrogen atom or a lower alkyl, lower alkanoyl, lower alkylsulfonyl, arylsulfonyl, loweralkoxyloweralkyl, 2,2,2 -trifluoroethyl or benzyl group;

and $Y^1$ and $Y^2$ are independently hydrogen or halogen atoms or hydroxy, trifluoromethyl, lower alkyl, lower alkoxy, nitro, unsubstituted amino, monoloweralkylamino, di-loweralkylamino, loweralkanoylamino, loweralkylsulfonylamino, arylsulfonylamino, N-loweralkyl-N-loweralkanoylamino or N-loweralkyl-N-loweralkylsulfonylamino groups;

and the pharmaceutically acceptable acid addition salts thereof;

the lower alkyl, lower alkoxy and lower alkanoyl groups containing from 1 to 6 carbon atoms.

2. A compound as claimed in claim 1 wherein Alk is an acyclic alkylene group, said compound preferably having the formula

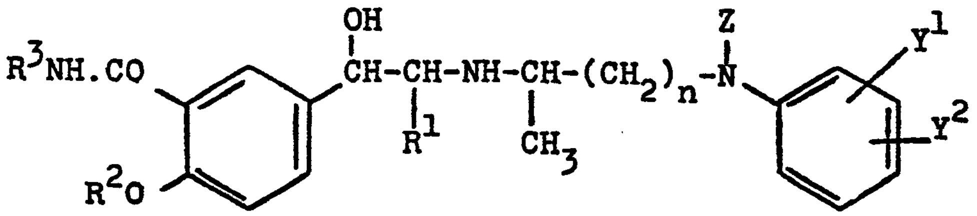

(II)

wherein n is 1-4, preferably 2, and $R^1$, $R^2$, $R^3$, Z, $Y^1$ and $Y^2$ are as defined in claim 1.

3. A compound as claimed in claim 1 wherein Alk is a cyclic alkylene group, said compound preferably having the formula

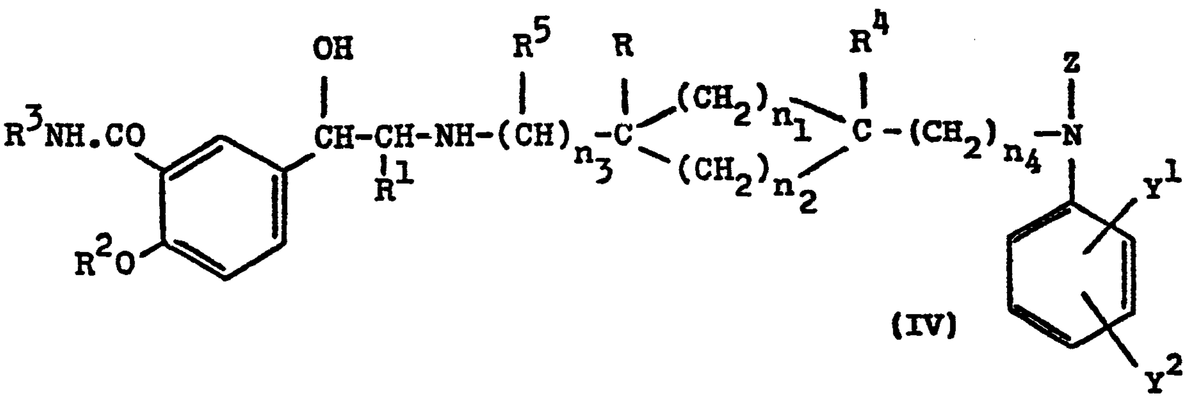

(IV)

wherein $\underline{n}_1$ is 0-2,

$\underline{n}_2$ is 1 - 5,

$\underline{n}_1 + \underline{n}_2$ is 2 - 6,

$\underline{n}_3$ and $\underline{n}_4$ are independently 0 or 1,

R, $R^4$ and $R^5$ are independently hydrogen atoms or lower alkyl groups with the proviso that the total number of carbon atoms in the Alk bridge does not exceed ten,

and $R^1$, $R^2$, $R^3$, Z, $Y^1$ and $Y^2$ are as defined in claim 1.

4. A compound as claimed in any of claims 1 to 3 wherein $R^2$ and $R^3$ are hydrogen atoms, $R^1$ is a hydrogen atom or a methyl group, and Alk, Z, $Y^1$ and $Y^2$ are as defined in claim 1; in particular wherein $R^1$, $R^2$, $R^3$ and $Y^1$ are hydrogen atoms, $Y^2$ is a methyl group or a hydrogen or halogen atom, Z is a hydrogen atom or a methyl or benzyl group and Alk is a trimethylene, tetramethylene or 1,4-cyclohexylene group or a group of the formula

$$-CH(CH_3).(CH_2)_n-$$

where $\underline{n}$ is 1 - 4.

5. 5-{2-[3-(N-methylanilino)-2-propylamino]-1-hydroxyethyl} salicylamide,

5- {2-[5-(N-methylanilino)-2-pentylamino]-1-hydroxyethyl} salicylamide,

5- { 2-[4-(N-methylanilino)-2-butylamino]-1-hydroxyethyl} salicylamide,

5- { 2-[4-(4,N-dimethylanilino)-2-butylamino]-1-hydroxyethyl} salicylamide,

5- { 2-[4-(4-chloro-N-methylanilino)-2-butylamino]-1-hydroxyethyl} salicylamide , and

5- { 2-[4-(4-fluoro-N-methylanilino)-2-butylamino]-1-hydroxyethyl} salicylamide;

and their pharmaceutically acceptable acid addition salts.

6 A compound as claimed in any of claims 1 to 5 in the form of an acid addition salt with sulfuric, phosphoric, hydrochloric, hydrobromic, sulfamic, citric, lactic, oleic, succinic, tartaric, cinnamic, acetic, benzoic, gluconic or ascorbic acid.

7 A process for the preparation of a compound of the formula I defined in claim 1 or a pharmaceutically acceptable acid addition salt thereof, which comprises reducing a compound of the formula

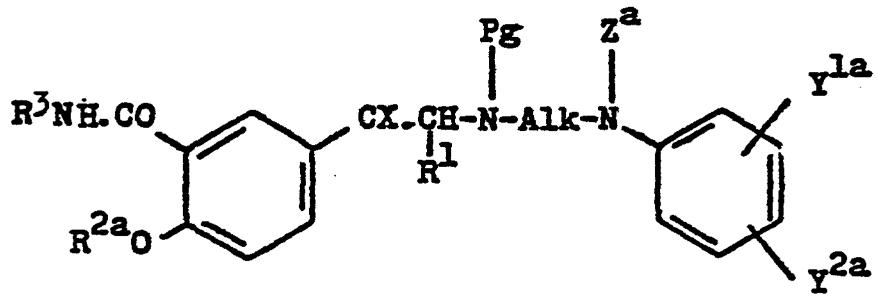

(V)

wherein X is an oxygen atom or (H,OH), Alk, $R^1$ and $R^3$ are as defined in claim 1, Pg is a hydrogen atom or a reductively removable protecting group, $R^{2a}$ and $Z^a$ are respectively $R^2$ and Z defined in claim 1 or a reductively removable protecting group, and $Y^{1a}$ and $Y^{2a}$ are $Y^1$ and $Y^2$ defined in claim 1, or a hydroxy or amino group (including unsubstituted and mono-substituted amino groups, but excluding di-substituted amino groups, defined in claim 1 for $Y^1$ or $Y^2$) carrying a reductively removable protecting group; provided that, when $R^{2a}$, Pg, $Z^a$, $Y^{1a}$ and $Y^{2a}$ are respectively the groups $R^2$, H, Z, $Y^1$ and $Y^2$, then X is an oxygen atom;

in an appropriate solvent ;

in particular a process wherein X is an oxygen atom and the reduction of the group CX to the group CHOH is effected by a borohydride in a water-miscible alcohol as solvent, especially by sodium borohydride in methanol, ethanol or isopropanol;

or a process wherein X is an oxygen atom and/or at least one reductively removable protecting group is present in $Y^{1a}$ and $Y^{2a}$ or as $R^{2a}$, Pg and $Z^a$, with the proviso that neither $Y^1$ nor $Y^2$ is a nitro group and Z is not a benzyl group, and the reduction is effected by means of hydrogen and a catalyst in the presence of an organic solvent, especially by means of hydrogen and palladium in a lower alkanol;

whereafter said compound of the formula I is isolated as such or in the form of a pharmaceutically acceptable acid addition salt thereof.

8. A process as claimed in claim 7 wherein a compound of the formula

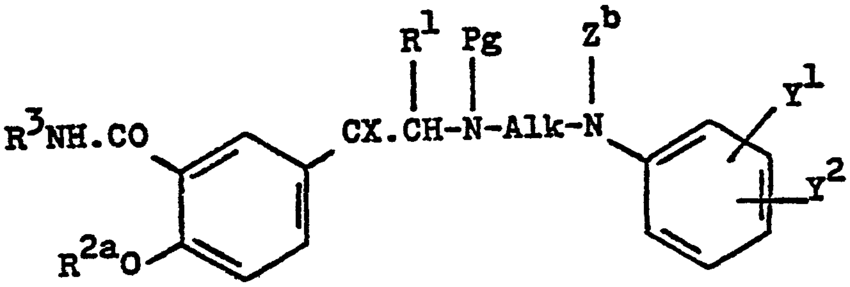

(VI)

wherein $R^1$, $R^3$, Alk, $Y^1$ and $Y^2$ are as defined in claim 1, $Z^b$ is the group Z defined in claim 1 other than a benzyl group, and X, $R^{2a}$ and Pg are as defined in claim 7, with the proviso that, when $R^{2a}$ and Pg are $R^2$ and a hydrogen atom respectively, then X is an oxygen atom, is reduced.

9. A process for the preparation of a compound of the formula IA:

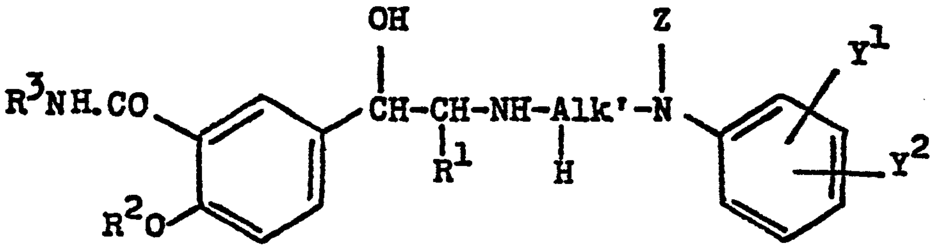

(IA)

or a pharmaceutically acceptable acid addition salt thereof,

wherein $R^1$, $R^2$, $R^3$, $Y^1$, $Y^2$ and Z are as defined in claim 1 and $\underset{\mathrm{H}}{\mathrm{Alk'}}$ is an acyclic or cyclic alkylene bridge as defined in claim 1 for Alk with the proviso that the designated hydrogen atom in $\underset{\mathrm{H}}{\mathrm{Alk'}}$ and the nitrogen atom of the ethanolamine moiety $-CHOH.CHR^1-$ are bonded to the same carbon atom,

which comprises the reductive condensation of a compound of the formula

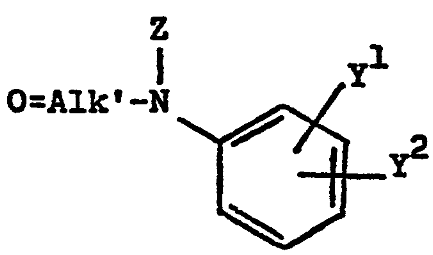

(IX)

wherein Alk' is as defined above and $Y^1$, $Y^2$ and Z are as defined in claim 1, with a compound of the formula

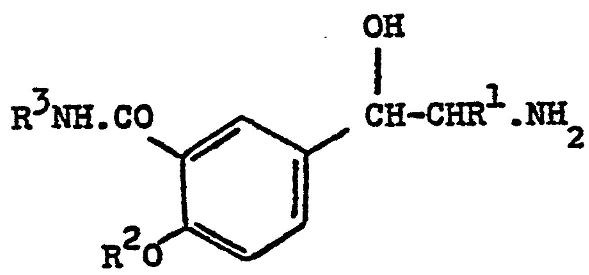

(X)

wherein $R^1$, $R^2$ and $R^3$ are as defined in claim 1, in the presence of an organic solvent;

in particular a process wherein the reduction is effected by means of catalytic hydrogenation, or lithium thexyllimonyl borohydride, or 9-borobicyclo[3,3,1]nonane, or especially by means of sodium borohydride or sodium cyanoborohydride in methanol, ethanol or propanol; whereafter said compound of the formula I is isolated as such or in the form of a pharmaceutically acceptable acid addition salt thereof.

10. A process as claimed in claim 9 wherein a compound of the formula II defined in

claim 2 is prepared by reductive condensation of a compound of the formula

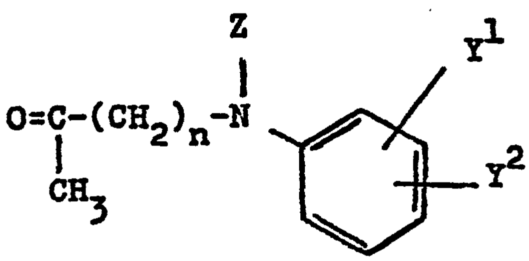

(IXA)

with a compound of the formula X defined in claim 9, wherein $R^1$, $R^2$, $R^3$, $Y^1$, $Y^2$, Z and n are as defined for formula II in claim 2.

11. Pharmaceutical compositions containing as active ingredient at least one compound or salt claimed in any of claims 1 to 6 together with a pharmaceutical carrier or excipient, especially such compositions in the form of dosage units such as tablets, capsules or injectable preparations in ampoules, which preferably contain from 2 to 500 mg. of active ingredient per dosage unit; or in the form of suspensions, syrups or elixirs.

0002867

European Patent Office

# EUROPEAN SEARCH REPORT

Application number
EP 78 20 0378

## DOCUMENTS CONSIDERED TO BE RELEVANT

| Category | Citation of document with indication, where appropriate, of relevant passages | Relevant to claim |
|---|---|---|
| | FR - A - 2 228 487 (ALLEN & HANBURYS)<br>* Claim 1; page 1 *<br>-- | 1,11 |
| | DE - A - 2 704 895 (ALLEN & HANBURYS)<br>* Claim 1; page 11 *<br>-- | 1,11 |
| | FR - A - 1 557 677 (ALLEN & HANBURYS)<br>* Page 1 *<br>---- | 1,11 |

CLASSIFICATION OF THE APPLICATION (Int. Cl.²)

C 07 C 103/85
A 61 K 31/165

TECHNICAL FIELDS SEARCHED (Int.Cl.²)

C 07 C 103/85
143/72

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

b The present search report has been drawn up for all claims

| Place of search | Date of completion of the search | Examiner |
|---|---|---|
| The Hague | 19-03-1979 | MOREAU |